# NEW EUROPEAN PATENT SPECIFICATION

(11) **EP 0 398 488 B2**
(45) Date of publication and mention of the opposition decision: **06.08.2003**
(45) Mention of the grant of the patent: 06.03.1996
(21) Application number: 90303738.0
(22) Date of filing: 06.04.1990
(51) Int. Cl.: A61N 1/365, A61N 1/368, A61N 1/39

(54) **Cardiac therapy device**
Herztherapie-Vorrichtung
Dispositif de thérapie cardiaque

(30) Priority: 19.05.1989 US 353967; 06.07.1989 US 376372
(43) Date of publication of application: 22.11.1990
(73) Proprietor: VENTRITEX, INC., Sunnyvale California 94086 (US)
(72) Inventor: Pless, Benjamin D., Menlo Park, California 94025 (US); Ball, Phillip L., San Jose, California 95129 (US); Fain, Eric, Menlo Park, California 94025 (US); Luceri, Richard M.D., FT. Lauderdale, Florida (US)
(74) Representative: MacGregor, Gordon

(56) References cited:
- EP-A- 0 060 117
- EP-A- 0 094 758
- EP-A- 0 253 505
- EP-A- 0 253 505
- US-A- 4 485 818
- PROCEEDINGS OF THE IEE 1989 CUSTOM INTEGRATED CIRCUITS CONFERENCE, San Diego, CA, 15th - 18th May 1989, pages 761-764; J.G. RYAN et al.: "A four chip implantable defibrillator/pacemaker chipset"

## Description

### FIELD OF THE INVENTION

The present invention concerns a novel device for cardiac therapy using an implanted cardiac pacer/defibrillator.

### BACKGROUND OF THE INVENTION

Implantable defibrillators are known in the prior art in which the spectrum of heart rates is divided into several distinct bands. Such bands may include normal sinus rhythm, tachycardia and fibrillation.

We have found that it would be extremely useful to divide the spectrum of heart rates into five bands, including bradycardia, normal sinus rhythm, slow tachycardia, fast tachycardia and fibrillation. However, when rhythms occur that have a combination of intervals, i.e., where the rhythm is oscillating around a border between two tachyarrhythmias, the implanted device must decide which arrhythmia is present. Further, the device must detect sinus rhythm when it occurs.

The present invention concerns a combined antitachycardia pacemaker/defibrillator which is implanted to treat potentially lethal arrhythmias. However, antitachycardia pacing can be an unpredictably prolonged therapy, and can result in leaving the patient in jeopardy if more definitive therapy is not used within a short period of time. One solution to this problem would be to severely limit the programability of the antitachycardia pacing feature. However, this is not a desirable solution.

Another potential problem in an automatic tiered therapy defibrillator, is that if a rapid arrhythmia such as a high rate tachycardia or fibrillation is detected, and the associated therapy is delivered, the device must know when to stop delivering the therapy. The device could be programmed to stop delivering therapy when the original detection criteria are no longer met.

Currently available defibrillators have a single rate cutoff. If it is exceeded, therapy is delivered. Normally if the average heart rate is high enough to be considered a tachycardia, an automatic device will provide tachycardia therapy. However, it is possible to have rhythms that have an average tachycardia rate but have an alternating pattern of intervals and should not be treated. These rhythms are sometimes called bigeminal rhythms. An example of a bigeminal rhythm is 600msec/300msec/600msec/300msec/etc. The average interval is 450msec. If the tachycardia detection criterion is 500msec, the device would inappropriately diagnose tachycardia.

Some prior art antitachycardia devices require a sequential number of intervals below the interval criterion for tachycardia. However, this makes it difficult to detect arrhythmias unless the arrhythmia rate is very stable.

In US-4485818 there is described a microprocessor controlled cardiac pacer which is capable of entering a temporary pacing mode for the treatment of tachyarrhythmia, which mode is exited after a predetermined period of normal atrial activity.

In EP-0094758 there is described a microprocessor controlled cardiac pacer which detects the existence of tachyarrhythmia by measuring the average time interval between successive heart beats and for comparing a sample beat with both a percentage of this average, and with a preset time interval.

In EP 0 253 505 there is disclosed a cardiac stimulator for detecting and treating venticular tachyarrhythmias that includes means for selectively dividing the heart rate continuum into progressively higher heart rate ranges.

Another object of the present invention is to provide definitive therapy for patients suffering from tachycardia, using an implanted combined antitachycardia pacemaker/defibrillator.

A still further object of the present invention is to provide arrhythmia detection inhibition with low tachycardia rate averages, but interval alternans. In this manner, the system will keep track of the ratio of sinus intervals to tachycardia intervals, and will require more tachycardia intervals than sinus intervals in order for an arrhythmia to be detected. Therefore the presence of a tachycardia can be quickly determined, without inappropriately detecting a bigeminal rhythm as tachycardia.

Other objects and advantages of the present invention will become apparent as the description proceeds.

### SUMMARY OF THE INVENTION

The present invention provides a cardiac pulse generator according to claim 1. In one embodiment, the device comprises means for: sensing a patient's heartbeat; providing storage means including a plurality of temporary storage bins, each of which corresponds to a different cardiac rhythm band; determining the intervals between heartbeats; incrementing the storage bin corresponding to the cardiac rhythm band of the determined heartbeat interval weighted by the current average; assigning a maximum count limit to each storage bin; detecting when the first bin reaches its maximum count limit; providing a diagnosis of the patient's cardiac rhythm that is responsive to the first bin to reach its maximum count limit; and initializing the temporary storage bins.

In the illustrative embodiment, the means for providing storage means comprises means for providing a sinus storage bin, a low rate tachycardia storage bin, a high rate tachycardia storage bin, and a fibrillation storage bin.

The device of an embodiment in accordance with the principles of the present invention also comprises means for: sensing a patient's heartbeat; determining the intervals between heartbeats; upon detection of a tachycardia, starting a duration timer to time a predetermined time period and treating for tachycardia in accordance with a programmed routine; if sinus is detected during the predetermined time period, then dearing the duration timer; if fibrillation is detected during the predetermined time period, then clearing the duration timer and treating for fibrillation; and if tachycardia continues for the predetermined time period, then commencing fibrillation therapy.

In the illustrative embodiment, the means for determining the intervals between heartbeats includes means for averaging a selected number of heartbeats.

The device of an embodiment of the present invention also comprises means for: sensing a patient's heartbeat; determining the intervals between heartbeats; if the heartbeats exceed a first rate, then treating for an arrhythmia; and continuing to treat for an arrhythmia unless the heartbeat rate declines to below a second rate, with the second rate being lower than the first rate. This allows use of a higher arrhythmia detection rate in a tiered defibrillator, without the disadvantage of failing to terminate slower arrhythmias that might result from the therapy.

The device of an embodiment of the present invention also includes means for: sensing a patient's heartbeat, determining the intervals between heartbeats; determining the ratio of sinus intervals to arrhythmia intervals; treating for arrhythmia only if the number of arrhythmias exceed the number of sinus intervals notwithstanding that the average of the sinus and arrhythmia intervals is shorter than sinus rhythm. This prevents the system from inappropriately detecting a bigeminal rhythm as an arrhythmia.

A more detailed explanation of the invention is provided in the following description and claims, and as illustrated in the accompanying drawings.

### BRIEF DESCRIPTION OF THE DRAWINGS

Fig. 1 is a block diagram of an implantable pacer/defibrillator system constructed in accordance with the principles of the present invention.
Fig. 2 is a flow chart of the arrhythmia discrimination method of the system of Fig. 1.
Fig. 3 is a flow chart of the bin interval block from the flow chart of Fig. 2.
Fig. 4 is a flow chart of the determine rhythm block from the flow chart of Fig. 2.
Fig. 5 is a flow chart of the interval alternans diamond from the flow chart of Fig. 2.

### DETAILED DESCRIPTION OF THE ILLUSTRATIVE EMBODIMENT

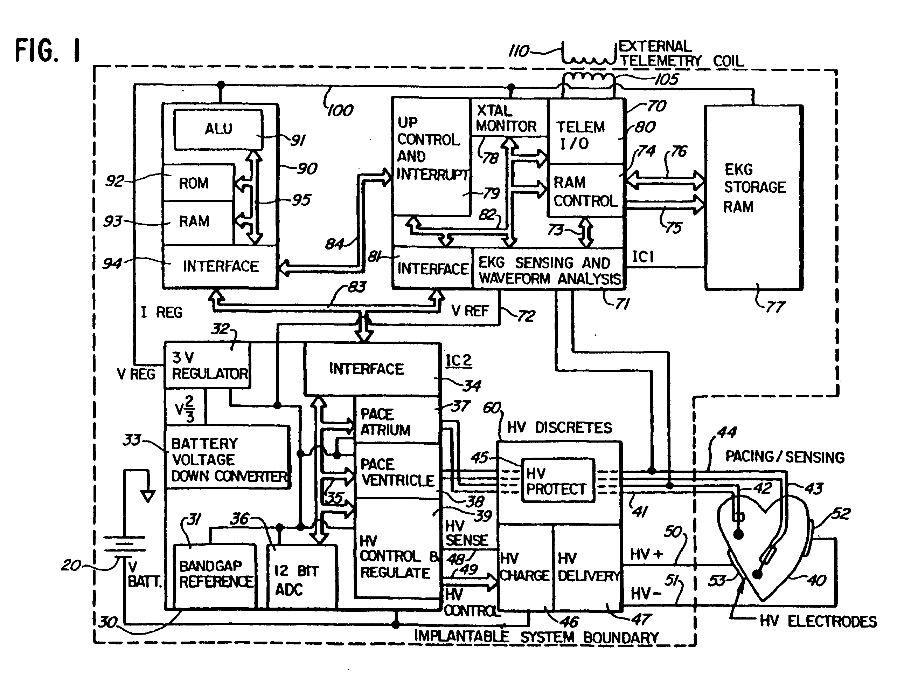

Referring to Figure 1, the block diagram for the implantable defibrillator includes four ICs and a set of high voltage discretes. The battery produces a positive voltage with respect to ground that varies from about 6.4 volts when new, to 5.0 volts at the end of service. The battery directly powers IC2 30 and the high voltage discretes 60.

IC2 contains a band-gap reference circuit 31 that produces 1.235 volts, and 3 volt regulator that powers the microprocessor 90, IC1 70, and the ECG storage RAM 77 through line 100. The 3 volt regulator runs off of a switched capacitor V 2/3 battery voltage down converter 33 for improved efficiency.

The microprocessor 90 communicates with IC2 through a data and address bus 83 and an on-chip interface 34 that contains chip-select, address decoding and data bus logic as is typically used with microprocessor peripherals. The internal bus 35 allows the microprocessor to control a general purpose ADC 36, the atrial pace circuits 37, the ventricular pace circuits 38, and the HV control and regulate block 39.

The ADC 36 is used by the microprocessor to measure the battery and other diagnostic voltages within the device.

The atrial pace circuits 37 indude a DAC that provides the ability to pace at regulated voltages. It communicates with the atrium of a heart 40 through two lines. One line 41 is a switchable ground; the other line 42 is the pacing cathode and is also the input to the atrial sense amplifier, as will be described below.

The ventricular pace circuits 37 include a DAC that provides the ability to pace at regulated voltages. It communicates with the ventricle of a heart 40 through two lines. One line 43 is a switchable ground; the other line 44 is the pacing cathode and is also the input to the ventricular sense amplifier, as will be described below.

Both the atrial and ventricular pace lines pass through high voltage protection circuits 45 to keep the defibrillation voltages generated by the device from damaging the pacing circuits 37 and 38.

The HV control and regulate block 39 on IC2 30 is used by the microprocessor 90 to charge a high voltage capacitor included in the HV charge block 46 to a regulated voltage, and then to deliver the defibrillating pulse to the heart 40 through the action of switches in the HV delivery block 47. An HV sense line 48 is used by the HV regulation circuits 39 to monitor the defibrillating voltage during charging. An HV control bus 49 is used by the HV control circuits 39 to control the switches in the HV delivery block 47 for delivering the defibrillating pulse to the electrodes 52, 53 through lines 50 and 51.

IC1 70 is another microprocessor peripheral and provides timing, interrupt, telemetry, ECG storage, and sensing functions.

A dual channel electrogram sensing and waveform analysis section 71 interfaces with the atrium and ventricle of the heart 40 through lines 42 and 44 respectively. The sensed electrogram is amplified and digitized. The amplifiers contained in this section 71 have multiple gain settings that are under microprocessor control for maintaining an AGC. Features such as peak voltage and complex width are extracted by the waveform analysis circuits 71 for the microprocessor 90 to use in discriminating arrhythmias from normal sinus rhythm. The voltage reference 31 from IC2 30 is used by the digitizer circuit 71 in the usual fashion, and is supplied by line 72.

The digitized ECG is provided to the RAM controller 74 through a bus 73. The RAM controller sequences through the addresses of a static RAM 77 to maintain a pretrigger area, and this produces a post trigger area upon command from the microprocessor 90.

The crystal and monitor block 78 has a 100KHz crystal oscillator that provides clocks to the entire system. The monitor is a conventional R-C oscillator that provides a back-up clock if the crystal should fail.

The microprocessor communicates with IC1 through two buses, 83 and 84. One bus 83 is a conventional data and address bus and goes to an on-chip interface 81 that contains chip select, address decoding and data bus drivers as are typically used with microprocessor peripherals. The other bus 84 is a control bus. It allows the microprocessor to set up a variety of maskable interrupts for events like timer timeouts, and sense events. If an interrupt is not masked, and the corresponding event occurs, an interrupt is sent from IC1 70 to the microprocessor 90 to alert it of the occurrence. On IC1 70, the up control and interrupt section 79 contains microprocessor controllable timers and interrupt logic.

The device can communicate with the outside world through a telemetry interface 80. A coil 105 is used in a conventional fashion to transmit and receive pulsed signals. The telemetry circuits 80 decode an incoming bit stream from an external coil 110 and hold the data for subsequent retrieval by the microprocessor 90. When used for transmitting, the circuit 80 receives data from the microprocessor 90, encodes it, and provides the timing to pulse the coil 105. The communication function is used to retrieve data from the implanted device, and to change the modality of operation if required.

The microprocessor 90 is of conventional architecture comprising an ALU 91, a ROM 92, a RAM 93, and interface circuits 94. The ROM 92 contains the program code that determines the operation of the device. The RAM 93 is used to modify the operating characteristics of the device as regards modality, pulse widths, pulse amplitudes, and so forth. Diagnostic data is also stored in the RAM for subsequent transmission to the outside world. The Algorithmic Logic Unit (ALU) 91 performs the logical operations directed by the program code in the ROM.

The program code is written to perform certain desirable functions which are best described in flowchart form.

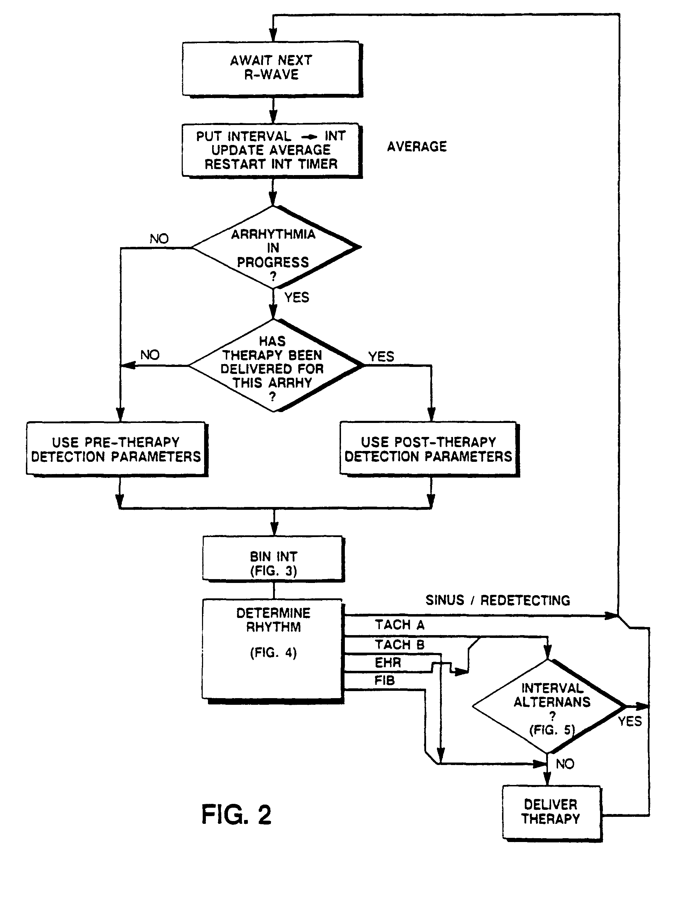

The method of arrhythmia discrimination is illustrated in the flow chart of Fig. 2. The "bins" referred to therein are RAM memory locations. The basic technique is to use the average of the last four sensed events to help decide how to bin each heartbeat interval. The first bin to reach its programmed number of counts determines the diagnosis.

The following technique rapidly converges on a solution. In the event that the arrhythmia is indeterminate, it defaults to the more conservative diagnosis.

Referring to Fig. 2, the system contains a loop that starts by awaiting the next R-wave. After the R-wave is detected, the interval of the R-wave is put into a register called INT. A register called AVERAGE is updated with that interval (AVERAGE is the average of the last four intervals), and the INT timer is restarted. The system then questions whether or not the arrhythmia is in progress. If no arrhythmia is in progress, the pre-therapy detection parameters are used. If the arrhythmia is in progress and therapy has already been delivered for this arrhythmia, then the post-therapy detection parameters are used.

In this embodiment, the post-therapy detection parameter is effectively a lower rate cutoff than the pre-therapy detection parameter. Thus if a high rate tachycardia is detected, there will be continued treatment for the tachycardia notwithstanding the fact that it may revert to a lower rate tachycardia. In other words, the post-therapy detection parameters will detect what normally would have been a low rate tachycardia as if it were a high rate tachycardia. Thus even if the arrhythmia slows to the range that would previously have been a low rate tachycardia, it is considered to be a high rate tachycardia episode. In this manner, the therapies will increase in effectiveness rather than going backwards in effectiveness.

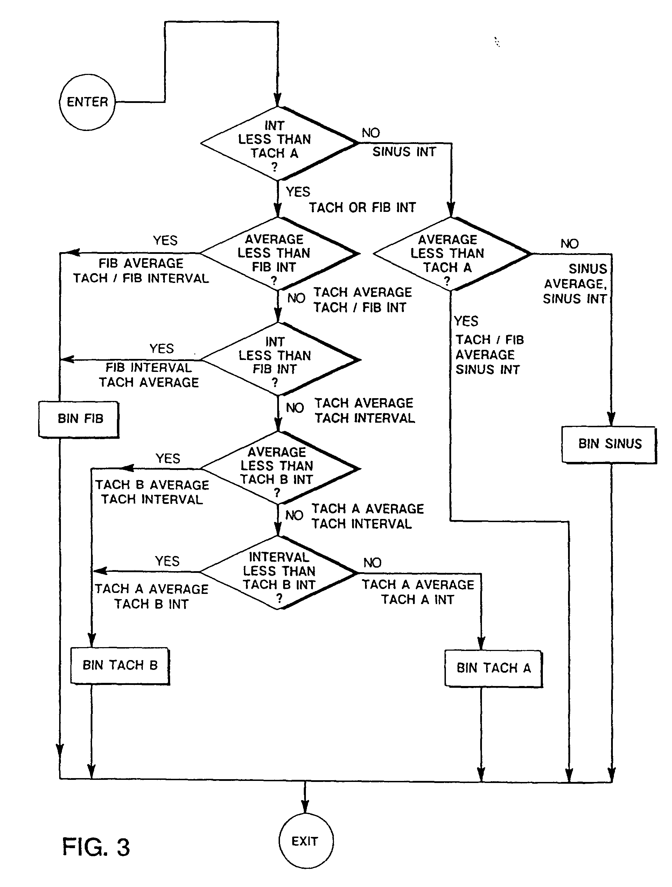

The system then falls through to the bin interval block. At that time, the system decides whether the interval that it just received belongs in low rate tachycardia (TACH A), high rate tachycardia (TACH B), fibrillation, or the sinus bin. The bin interval block is described in more detail below with respect to Fig. 3.

The bin interval block leads to the determine rhythm block where the results of the binning exit as either having detected sinus, low rate tachycardia, high rate tachycardia, EHR (extended high rate) or having detected fibrillation.

If the system detects EHR or low rate tachycardia, an additional check is made to see if the interval alternans inhibition is in effect. In this manner, the system looks for the case where there is a tachycardia/sinus/tachycardia/sinus, etc. sequence of events. If that is the case, the system inhibits delivering therapy and waits for the next R-wave. If that is not the case, or if the system detects high rate tachycardia or fibrillation, then it falls through to delivering therapy. After delivering therapy, the system returns to waiting for the next R-wave.

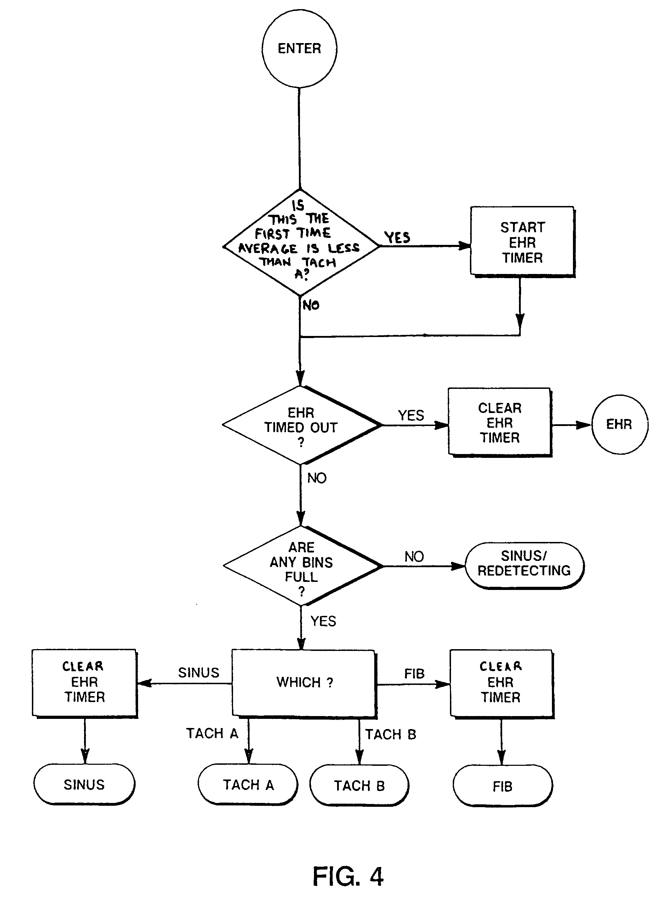

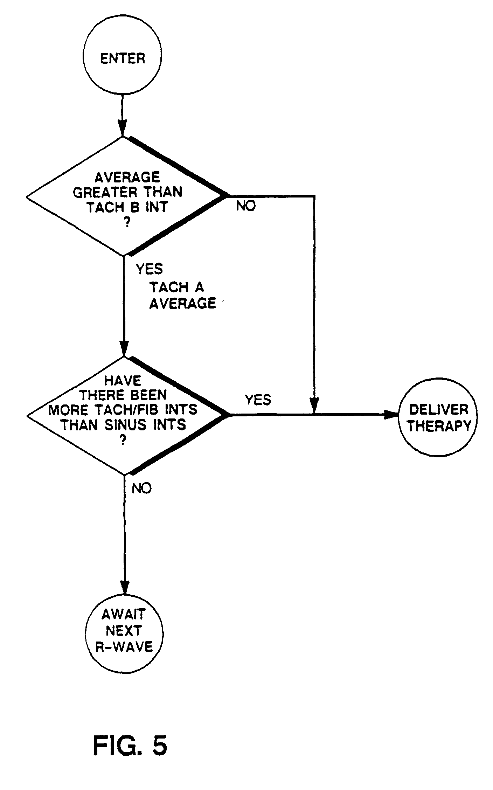

The "determine rhythm" block is described in more detail with respect to Fig. 4, and the interval alternans block is described in more detail below with respect to Fig. 5, described below.

In summary with respect to the discrimination flow chart of Fig. 2, the patient's R-waves are sensed and put into an interval storage register labeled INT. There is a short term averaging of the intervals (preferably, the last four intervals are averaged). Depending upon the average interval, it can then be determined into which bin the interval should be put.

Now referring to the bin interval flow chart of Fig. 3, the flow chart is entered from the detection hysteresis part of the flow chart of Fig. 2, where the system determines whether to use pre-therapy detection parameters or the post-therapy detection parameters. Those parameters are the rates at which the system detects the tachycardia and the number of intervals required for detecting tachycardia. The number of intervals required is the depth of the bin. First, the system checks whether the interval is shorter than low rate tachycardia (TACH A). If so, that would mean that the interval would be a tachycardia or fibrillation interval. If it is longer than low rate tachycardia (TACH A), then it is a sinus interval and the system exits.

The average interval (AVERAGE) is then checked to see if it is shorter than a low rate tachycardia. If it is not, there is a sinus average and a sinus interval and the system bins one sinus count. If the average is less than a low rate tachycardia, that means the patient has a tachycardia or fibrillation average, but since the system detected a sinus interval, nothing is binned. The system just falls through to the exit.

If, at the first decision block, the system determines that the interval is less than TACH A, then the patient has a tachycardia or a fibrillation interval and the system checks to see whether AVERAGE is less than the fibrillation requirement. If AVERAGE is less than the fibrillation criterion, the system bins a fibrillation interval. Thus whether it is a TACH interval or a TACH A or a TACH B interval, the fact that AVERAGE is fibrillation means that the system will go ahead and bin it as a fibrillation interval.

If, on the second decision block through the main decision block tree, the system determines that AVERAGE is not less than the fibrillation interval, that would mean that AVERAGE is a tachycardia average since the system has already determined that it is not a fibrillation average, but the interval may either be a tachycardia interval or a fibrillation interval. The system then checks to see whether the interval is less than the fibrillation interval requirement. If it is, the system bins the interval as a fibrillation interval. If not, then the system is at the point where it recognizes that the patient has a tachycardia average and a tachycardia interval. The system then has to determine whether it is a low rate tachycardia or a high rate tachycardia. To this end, the system checks to see if the average is less than the high rate tachycardia interval. If it is, then the patient has a high rate tachycardia average and it is binned as a TACH B. If not, the patient has a low rate tachycardia average, and the system has to check to see if the interval is less than the TACH B interval. If it is, then it is binned as a TACH B interval. If it is not, then it is binned as a TACH A interval and exits.

It can be seen that, with respect to the bin interval system, the average interval (AVERAGE) is used to determine where to bin the interval. In addition, the system checks to determine whether the interval represents sinus, TACH A, TACH B or fibrillation. As different intervals are detected, the decision blocks place the interval data into different bins depending on the interval determination using AVERAGE. Until a bin is full, the system has not decided what the arrhythmia is. Once the bin is filled, the system then diagnoses sinus rhythm or an arrhythmia and treats for that particular arrhythmia in response to the filled bin.

In a specific example, the values that the microprocessor uses to implement arrhythmia detection are as follows. These are fixed values that can be adjusted to change the arrhythmia detection for individual patients. The ranges and nominal values are shown for information only.
- TACH A INT: Intervals between this and TACH B INT are used to diagnose TACH A. Intervals longer than this are considered sinus; 450 msec.
- TACH B INT: Intervals between this and FIB INT are used to diagnose TACH B; 380 msec.
- FIB INT: Intervals shorter than this are used to diagnose fibrillation; 310 msec.
- NUM SINUS: The number of intervals for sinus detection; 3.
- NUM TACH: A The number of intervals for TACH A detection; 8.
- NUM TACH: B The number of intervals for TACH B detection; 8.
- NUM FIB: The number of intervals for fib detection; 12.
The microprocessor maintains the following registers:
- AVERAGE: The average of the last four intervals.
- SINUS CNT: The number of sinus intervals detected.
- TACH A CNT: The number of TACH A intervals detected.
- TACH B CNT: The number of TACH B intervals detected.
- FIB CNT: The number of fib intervals detected.
- TS RATIO: Used to avoid detecting a rhythm with interval alternans as an arrhythmia.
AVERAGE is the average of the last four ECG intervals and is updated by the microprocessor every interval. Every interval is compared against AVERAGE. The intervals are binned by the microprocessor according to the following conditions: 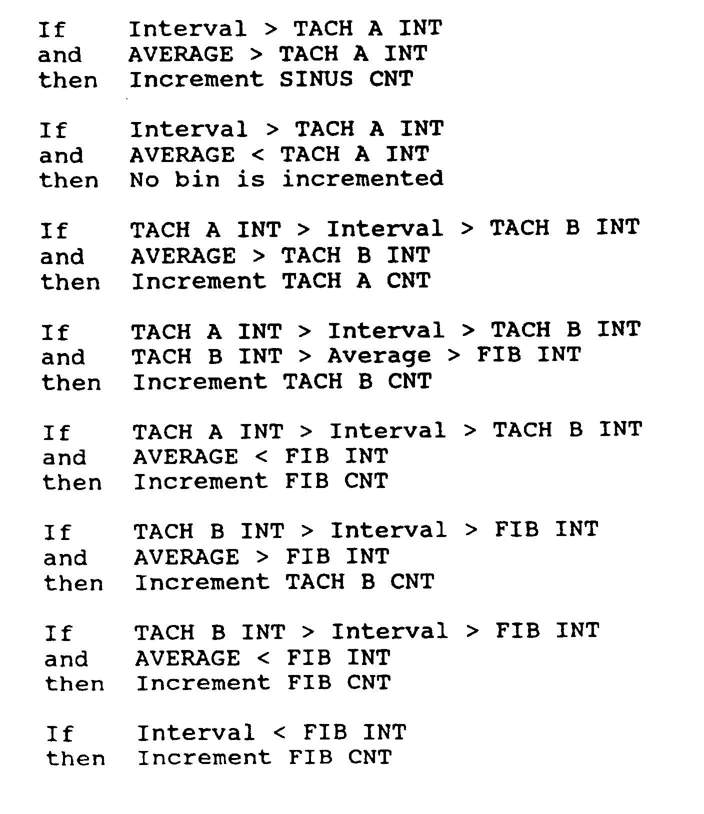 The microprocessor increments the appropriate bin after every detected ECG Interval. The first bin to count up to its respective limit causes that arrhythmia, or sinus rhythm, to be diagnosed. Upon each diagnosis, the arrhythmia detection bins are initialized.

In the operation of the system, if the system sees mixed TACH A and TACH B intervals is AVERAGE is TACH B, all the intervals are binned as TACH B. Therefore, if there is a high rate tachycardia average, the presence of some slow intervals will not cause the system to delay deciding on an arrhythmia diagnosis; the arrhythmia interval will still be binned the same as if they were the faster arrhythmia. If there is a fibrillation average, then any TACH interval gets binned as a fibrillation. If the system detects a TACH B average, then any TACH A interval gets binned as a TACH B. In this manner, the system defaults to the most conservative diagnosis. For example, if the average is TACH B and a fibrillation interval is detected, the system will bin a fibrillation interval rather than a TACH B interval in order to be as conservative as possible. In summary, the system always tends to bin diagnose the more serious arrhythmia.

The following is a discussion of the determine rhythm flow chart of Fig. 4. Referring to Fig. 4, once the interval has been placed into a bin, the system must determine whether an arrhythmia has been detected. In effect, the system is determining whether a bin is full. However, the system must first go through and see if this is a first time that AVERAGE is less than TACH A. If it is, that means that the patient has gone from either sinus to the beginning of a tachycardia or there has been a defibrillation therapy but there is still a tachycardia in progress. If either one of those are true, then the extended high rate (EHR) timer is started. If the EHR timer has been ongoing, the system just falls through and checks to see if the EHR timer has timed out. If it has, the EHR timer is deared and the diagnosis is that EHR is the arrhythmia that has been detected.

If the EHR timer has not timed out, the system falls through and checks to see if any of the bins are full. If none of the bins is full, the system is either redetecting sinus or is redetecting one of the arrhythmias and the state is indeterminate. The system goes to the redetecting exit. If a bin is full, since only one bin will be full at this time, the system checks to see which bin is filled and then clears all of the bins.

If the system detects that either a sinus bin is full or a fibrillation bin is full, then the EHR timer is cleared and the diagnosis is either sinus or fibrillation, respectively. If the system determines either a TACH A or a TACH B, the EHR timer is not cleared and the diagnosis is TACH A or TACH B, respectively. The depth of a bin is determined in memory by how the device is programmed. Thus, each bin is a RAM location that is counting up to a value that is determined by another RAM location that is under programmer control. The programmer can program the particular interval that is used to determine whether or not it is a TACH A, a TACH B or a fib interval. It is those programmable parameters, i.e., the interval cutoff and the depth of the bin, that is used in the implementation of detection hysteresis. Thus there are two parameters per arrhythmia.

Typical examples for those parameters are set forth above. For example, TACH A may be set at 450 msec., TACH B at 380 msec., and fibrillation at 310 msec. Thus, if the system detects an interval that is between 450 msec. and 380 msec., it would be binned as a TACH A interval if the average were also TACH A. If the interval were between 380 msec. and 310 msec., it is a TACH B interval if the average is TACH B or TACH A. If the interval is shorter than 310 msec., then it is binned as a fibrillation interval. If the interval is longer than 450 msec., then it is a sinus interval and the number of intervals required, may be 3 to detect sinus, 8 to detect TACH A, 8 to detect TACH B and 12 to detect fibrillation. This means that the fibrillation bin is 12 deep, the sinus bin is 3 deep, etc. The bins, however, can be programmable up to 255 intervals but the previous numbers are being given as specific examples.

As a further explanation of the extended high rate (EHR) system, as soon as the average goes from being a sinus average to a TACH average, the EHR timer is started. Then, if the EHR timer times out at any time, whether the bins are full or not, that determines that the system has detected EHR. EHR is another way to start fibrillation or some other programmed therapy. If the system detects sinus at any time, or if it detects fibrillation or EHR at any time, the EHR timer is cleared.

The following is an example of what will happen if any arrhythmia starts the EHR timer. If the arrhythmia does not persist, and sinus rhythm is detected, then the EHR timer is cleared until another arrhythmia starts. The arrhythmia starts, TACH A is detected, some TACH A therapy is given, and possibly TACH B is detected and some TACH B therapy is given. The EHR timer then times out, and the system will abandon the TACH A or TACH B therapy and will revert to fibrillation therapy. In other words, it is basically a safety exit to prevent the patient from being engaged in less effective therapies for a long time.

The interval alternans flow chart of Fig. 5 will now be discussed.

The interval alternans is entered if the determine rhythm block shows either a TACH A or an EHR. In that case, the average is checked to see if it is greater than a TACH B average. If it is not greater than a TACH B average, then the rhythm is rapid. Thus it is either a TACH B average or a fibrillation average and in that case therapy is delivered whether or not there is an interval alternans.

If, however, the average is greater than a TACH B interval, that means that the patient has a TACH A average and then a further check is made to see whether there have been more TACH intervals than sinus intervals during the detection period. If there have not, then the system determines that it is not an arrhythmia and awaits the next R-wave. If there have been more TACH or fibrillation intervals than sinus intervals, the system continues on to deliver the therapy. The reason for this is that if there is a bigeminal rhythm, the average rate might be a TACH A rate but the patient is not in arrhythmia and it is desirable to avoid treating the patient, even though the short intervals may be TACH B intervals or fibrillation intervals. This interval alternans program provides a means to avoid treating a bigeminal rhythm.

A novel device for cardiac therapy has been shown and described. By means of the present invention, the system can rapidly converge to a solution and in the event that the arrhythmia is indeterminate, there is a default to the most conservative diagnosis. By using a novel method of binning, the system allows the use of a higher arrhythmia detection rate in a tiered defibrillator, without the disadvantage of failing to terminate slower arrhythmias that might result from the therapy. The invention also allows the system to keep track of the ratio of sinus intervals to tachycardia intervals, and to require more tachycardia intervals than sinus intervals so that a bigeminal rhythm will not be detected as a tachycardia.

Although a detailed embodiment of the invention has been shown and described, it is to be understood that various modifications and substitutions may be made by those skilled in the art without departing from the scope of the invention as defined by the appended claims. For example, the "incrementing" of the storage bins could be negative incrementation, i.e. decrementing from a certain level. In addition, the "bins" referred to could be any storage media, digital or analog, and the "counts" could comprise analog levels such as voltage levels or the like.

## Claims

1. An implantable cardiac pulse generator comprising:
means (71) for sensing a patient's heartbeat;
means (90) for determining the intervals between heartbeats;
storage means (93) including a plurality of storage count bins, each of the storage count bins corresponding to a different cardiac rhythm band;
means (92) for assigning a count limit to each storage bin;
means (90) for detecting when a first bin reaches its count limit;
means (90) for providing a diagnosis of the patient's cardiac rhythm that is responsive to the first bin to reach its count limit;
means (90) for initialising said plurality of storage bins upon said diagnosis of a cardiac rhythm; and
means (90) for changing the count of the storage bin corresponding to the cardiac rhythm band of the determined heartbeat interval, the plurality of storage bins simultaneously maintaining cumulative counts corresponding to each of the different cardiac rhythm bands between each diagnosis event.

2. An implantable cardiac pulse generator according to claim 1 wherein the plurality of storage count bins includes a sinus bin, a tachycardia bin and a fibrillation bin.

3. An implantable cardiac pulse generator as defined by claim 1 wherein the means (92) for assigning the count limit to each bin includes means for assigning different count limits to different bins.

4. An implantable cardiae pulse generator as defined by claim 1 wherein the means (90) for determining the intervals between heartbeats includes means for averaging a predetermined number of sensed heartbeats to provide an average interval.

5. An implantable cardiac pulse generator as defined by claim 2 wherein the plurality of storage bins includes a low rate tachycardia bin and a high rate tachycardia bin.

6. An implantable cardiac pulse generator according to claim 2 further including:
means (90) for tracking the ratio of sinus intervals to tachycardia intervals; and
means (41 - 44, 60) for treating for tachycardia if there are more tachycardia intervals than sinus intervals but not treating for tachycardia if there are more sinus intervals than tachycardia intervals.

7. A cardiac therapy device comprising the implantable cardiac pulse generator according to claim 1 and further comprising:
means (41 - 44, 60) for treating for an arrhythmia if the heartbeats exceed a first rate; and
means (41 - 44, 60) for continuing to treat for an arrhythmia unless the heartbeat rate declines to below a second rate, with the second rate being lower than the first rate.

8. A cardiac therapy device comprising an implantable cardiac pulse generator according to claim 1 and further including:
means (50 - 53, 60) for applying a high energy shock to the patient's heart;
a duration timer (70, 90) for timing a predetermined time period upon detection of a tachycardia;
means (90) for clearing the duration timer if sinus is detected during said predetermined time period; and
means (90) for clearing the duration timer and treating for fibrillation if fibrillation is detected during said predetermined time period.

9. A cardiac therapy device as defined by claim 8 further including:
means for commencing fibrillation therapy if a tachyarrhythmia other than fibrillation continues for said predetermined time period.

10. A cardiac therapy device as claimed by claim 9 wherein said means (90) for determining the intervals between heartbeats includes means for averaging a selected number of intervals.

## Patentansprüche

1. Implantierbarer Herzpulsgenerator mit:
einer Einrichtung (71) zum Abtasten eines Herzschlags eines Patienten,
einer Einrichtung (90) zum Bestimmen der Intervalle zwischen Herzschlägen,
einer Speichereinrichtung (93) einschließlich einer Mehrzahl von Speicherzählabschnitten, wobei jeder der Speicherzählabschnitte einem unterschiedlichen Herzrhythmusband entspricht;
einer Einrichtung (92) zum Zuteilen einer Zählgrenze für jeden Speicherabschnitt,
einer Einrichtung (90) zum Detektieren, wann ein erster Abschnitt seine Zählgrenze erreicht,
einer Einrichtung (90) zum Bereitstellen einer Diagnose des Patientenherzrhythmus, die dem ersten seine Zählgrenze erreichenden Abschnitt entspricht,
einer Einrichtung (90) zum Initialisieren der Mehrzahl von Speicherabschnitten bei der Diagnose eines Herzrhythmus, und
einer Einrichtung (90) zum Ändern des Zählerstands des Speicherabschnitts entsprechend dem Herzrhythmusband des bestimmten Herzschlagintervalls, wobei die Mehrzahl von Speicherabschnitten gleichzeitig kumulative Zählstände entsprechend jedem der unterschiedlichen Herzrhythmusbänder zwischen jedem Diagnoseereignis unterhalten.

2. Implantierbarer Herzpulsgenerator nach Anspruch 1, bei dem die Mehrzahl von Speicherzählabschnitten einen Sinusabschnitt, einen Tachykardieabschnitt und einen Fibrillationsabschnitt umfasst.

3. Implantierbarer Herzpulsgenerator nach Anspruch 1, bei dem die Einrichtung (92) zum Zuteilen der Zählgrenze für jeden Abschnitt eine Einrichtung zum Zuteilen unterschiedlicher Zählgrenzen für unterschiedliche Abschnitte umfasst.

4. Implantierbarer Herzpulsgeherator nach Anspruch 1, bei dem die Einrichtung (90) zum Bestimmen der Intervalle zwischen Herzschlägen eine Einrichtung zum Mitteln einer vorbestimmten Anzahl an detektierten Herzschlägen umfasst, um ein Durchschnittsintervall bereitzustellen.

5. Implantierbarer Herzpulsgenerator nach Anspruch 2, bei dem die Mehrzahl von Speicherabschnitten einen Niedrigfrequenztachykardieabschnitt und einen Hochfrequenztachykardieabschnitt umfasst.

6. Implantierbarer Herzpulsgenerator nach Anspruch 2 ferner mit:
einer Einrichtung (90) zum Verfolgen des Verhältnisses der Sinusintervalle zu den Tachykardieintervallen, und
einer Einrichtung (41 bis 44, 60) zum Behandeln gegen Tachykardie, wenn mehr Tachykardieintervalle als Sinusintervalle vorhanden sind, jedoch zum Nichtbehandeln gegen Tachykardie, wenn mehr Sinusintervalle als Tachykardieintervalle vorhanden sind.

7. Herztherapievorrichtung mit dem implantierbaren Herzpulsgenerator nach Anspruch 1 und ferner mit:
einer Einrichtung (41 bis 44, 60) zum Behandeln einer Arhythmie, wenn die Herzschläge eine erste Frequenz übersteigen, und
einer Einrichtung (41 bis 44, 60) zum Fortsetzen einer Behandlung gegen Arhythmie, es sei denn, dass die Herzschlagfrequenz nicht unterhalb einer zweiten Frequenz sinkt, wobei die zweite Frequenz unterhalb der ersten Frequenz liegt.

8. Herztherapievorrichtung mit einem implantierbaren Herzpulsgenerator nach Anspruch 1 und mit weiterbin:
einer Einrichtung (50 bis 53, 60) zum Anlegen eines Hochenergieschocks an das Herz des Patienten,
einem Dauerzeitgeber (70, 90) zum zeitlichen Steuern einer vorbestimmten Zeitdauer bei Detektion einer Tachykardie,
einer Einrichtung (90) zum Löschen des Dauerzeitgebers, wenn ein Sinus während der vorbestimmten Zeitdauer detektiert wird, und
einer Einrichtung (90) zum Löschen des Dauerzeitgebers und Behandeln gegen Fibrillation, wenn eine Fibrillation während der vorbestimmten Zeitdauer detektiert wird.

9. Herztherapievorrichtung nach Anspruch 8 ferner mit:
einer Einrichtung zum Einleiten einer Fibrillationstherapie, wenn eine von der Fibrillation verschiedene Tachyarhythmie für die vorbestimmte Zeitdauer anhält.

10. Herztherapievorrichtung nach Anspruch 9, bei der die Einrichtung (90) zum Bestimmen der Intervalle zwischen Herzschlägen eine Einrichtung zum Mitteln einer ausgewählten Anzahl von Intervallen umfasst.

## Revendications

1. Générateur d'impulsions cardiaques, pouvant être implanté, comportant :
des moyens (71) pour détecter les battements du coeur d'un patient ;
des moyens (90) pour déterminer les intervalles entre battements du coeur ;
des moyens de mémorisation (93) comportant une pluralité de zones de mémorisation de comptage, chacune des zones de mémorisation de comptage correspondant à une bande de rythmes cardiaques différents ;
des moyens (92) pour assigner une limite de comptage à chaque zone de mémorisation ;
des moyens (90) pour détecter lorsqu'une première zone atteint sa limite de comptage ;
des moyens (90) pour fournir un diagnostic relatif au rythme cardiaque du patient, en réponse au fait que la première zone a atteint sa limite de comptage ;
des moyens (90) d'initialisation de ladite pluralité de zones de mémorisation sur la base dudit diagnostic du rythme cardiaque ; et
des moyens (90) sont agencés pour changer le comptage de la zone de mémorisation correspondant à la bande de rythmes cardiaques de l'intervalle déterminé de battements de coeur, la pluralité de zones de mémorisation maintenant simultanément des comptages cumulatifs correspondant à chacune des bandes de rythmes cardiaques différents entre chaque évènement de diagnostic.

2. Générateur d'impulsions cardiaques, pouvant être implanté, selon la revendication 1, dans lequel la pluralité de zones de mémorisation de comptage comportent une zone de sinus, une zone de tachycardie, et une zone de fibrillation.

3. Générateur d'impulsions cardiaques selon la revendication 1, dans lequel les moyens (92) d'assignation de la limite de comptage à chacune des zones comportent des moyens d'assignation de limites de comptage différentes à des zones différentes.

4. Générateur d'impulsions cardiaques selon la revendication 1 dans lequel les moyens (90) pour déterminer les intervalles entre battements du coeur comportent des moyens pour faire la moyenne d'un nombre prédéterminé de battements du coeur détectés pour fournir un intervalle moyen.

5. Générateur d'impulsions cardiaques selon la revendication 2, dans lequel la pluralité de zones de mémorisation comportent une zone de tachycardie de faible rythme et une zone de tachycardie de haut rythme.

6. Générateur d'impulsions cardiaques, pouvant être implanté, selon la revendication 2, comportant en outre :
des moyens (90) pour suivre le rapport des intervalles de sinus sur les intervalles de tachycardie, et
des moyens (41 à 44, 60) pour traiter la tachycardie s'il existe plus d'intervalles de tachycardie que d'intervalles de sinus mais ne pas traiter la tachycardie s'il existe plus d'intervalles de sinus que d'intervalles de tachycardie.

7. Dispositif de thérapie cardiaque comportant le générateur d'impulsions cardiaques, pouvant être implanté, selon la revendication 1 et comportant en outre :
des moyens (41 à 44, 60) pour traiter une arythmie si les battements du coeur dépassent un premier rythme, et
des moyens (41 à 44, 60) pour poursuivre le traitement de l'arythmie jusqu'à ce que le rythme des battements du coeur descende en dessous d'un second rythme, le second rythme étant inférieur au premier rythme.

8. Dispositif de thérapie cardiaque comportant un générateur d'impulsions cardiaques, pouvant être implanté, selon la revendication 1, et comportant en outre :
des moyens (50 à 53, 60) pour appliquer un choc d'énergie élevée au coeur du patient,
un dispositif de mesure de temps (70, 90) pour décompter une période de temps prédéterminée lors de la détection d'une tachycardie,
des moyens (90) pour remettre à zéro le dispositif de mesure de temps si un battement de sinus est détecté pendant ladite période de temps prédéterminée, et
des moyens (90) pour remettre à zéro le dispositif de mesure de temps et traiter une fibrillation si une fibrillation est détectée pendant ladite période de temps prédéterminée.

9. Dispositif de thérapie cardiaque selon la revendication 8, comportant en outre :
des moyens pour démarrer une thérapie de fibrillation si une tachyarythmie autre qu'une fibrillation se poursuit sur ladite période de temps prédéterminée.

10. Dispositif de thérapie cardiaque selon la revendication 9, dans lequel lesdits moyens (90) pour déterminer les intervalles entre battements du coeur comportent des moyens pour déterminer la moyenne d'un nombre choisi d'intervalles.
